# EUROPEAN PATENT SPECIFICATION

(11) **EP 1 778 205 B1**
(45) Date of publication and mention of the grant of the patent: **25.02.2009**
(21) Application number: 05763000.6
(22) Date of filing: 25.07.2005
(51) Int. Cl.: A61K 31/047, A61K 31/195, A61K 31/70, A61P 43/00

(54) **HYDRATING COMPOSITION**
HYDRATISIERENDE ZUSAMMENSETZUNG
COMPOSITION D'HYDRATATION

(30) Priority: 23.07.2004 US 590453 P; 23.07.2004 GB 0416493
(43) Date of publication of application: 02.05.2007
(73) Proprietor: THE UNIVERSITY COURT OF THE UNIVERSITY OF GLASGOW, Glasgow, Strathclyde G12 8QQ (GB)
(72) Inventor: PITSILADIS, Yannis Paul, Glasgow G11 6QE (GB)
(74) Representative: Forrest, Graham Robert
(86) International application number: PCT/GB2005/002913
(87) International publication number: WO 2006/008552

(56) References cited:
- US-A- 5 236 712
- US-A- 5 403 921
- US-A- 5 973 005
- US-A- 6 013 290
- PATENT ABSTRACTS OF JAPAN vol. 2003, no. 12, 5 December 2003 (2003-12-05) & JP 2004 123686 A (CRESCENDO CORPORATION), 22 April 2004 (2004-04-22)
- GREENWOOD MICHAEL ET AL: "Creatine supplementation during college football training does not increase the incidence of cramping or injury." MOLECULAR AND CELLULAR BIOCHEMISTRY, vol. 244, no. 1-2, February 2003 (2003-02), pages 83-88, XP002347973 ISSN: 0300-8177

## Description

### Field of the invention

The present invention relates to hydration of the body, and in particular to methods and compositions for enhancing the ability of the body to absorb ingested water and retain water in the tissues. Compositions and methods of the invention may be used for increasing physical and mental performance (e.g. athletic performance) in the heat, as well as for preventing and treating dehydration, particularly during prolonged exposure to high ambient temperatures.

### Background to the invention

Dehydration is one of the primary causes of fatigue during exercise in the heat, and also a major cause of heat-related illness. During a heat wave in 2003, an estimated 11,435 people in France died due to heat-related illness. During the 2003 Iraq conflict, allied soldiers had to drink at least 10 litres of water daily to avoid considerable dehydration.

Currently available rehydration solutions (e.g. sports drinks) contain water and electrolytes (mainly sodium, potassium and chloride) with the aim of actively replacing these components which are lost from the body in sweat. They also provide a limited amount of fuel, typically in the form of glucose. Replacing the electrolytes helps enhance the uptake of the ingested fluid and assists to retain water to a certain extent. Nevertheless, a considerable amount of the water will be lost in the urine and not actively retained.

In most circumstances, standard electrolyte-based rehydration solutions will fulfil most needs and hence they are widely used. However, in certain situations when water availability is restricted, or when rapid rehydration is paramount to prevent life-threatening dehydration (e.g. for endurance athletes and military personnel operating under high temperature conditions, as well as patients suffering from dehydration, such as those with severe diarrhoea), such solutions will be, to a large extent, ineffective.

There have been many attempts to hyperhydrate through the ingestion of fluids in order to minimise the deleterious effects of dehydration on exercise performance in the heat (Greenleaf & Castle, 1971; Moroff & Bass, 1965). However, the effectiveness of fluid replacement strategies is limited as any excess fluid consumed is filtered and excreted (Greenleaf & Castle, 1971). This has lead to experimentation with hyperhydration or "water-loading" agents such as glycerol with mixed success. Some studies have shown glycerol to be an effective hyperhydrating agent when ingested with a bolus of water (Gleeson *et al*. 1986; Hitchins *et al*. 1999; Lyons *et al*. 1990). This is achieved by reducing the rate of free water clearance, and increasing fluid retention by 400 to 700 ml after 2.5 to 4 hr of hydration involving 1.4 to 2.0 L of fluid (Robergs & Griffin, 1998). This water retention is evenly distributed throughout body water compartments (Robergs & Griffin, 1998). Some studies have demonstrated that the glycerol-induced increase in total body water (TBW) can attenuate the rise in both core temperature (Anderson *et al*, 2001; Lyons *et al*. 1990) and heart rate (Anderson *et al*. 2001; Montner *at al*. 1996) and improve endurance performance (Hitchins *et al*. 1999; Montner *at al*. 1996; Anderson *et al*. 2001) during exercise in the heat. However, other studies have failed to show that glycerol reduces thermal strain during exercise, or indeed influences exercise performance in the heat (Latzka *et al*. 1997; Latzka *et al*. 1998; Marino *et al*. 2003). This has lead to a number of alternative hyperhydrating methods being investigated.

US5973005 describes a beverage comprising creatine, glycerol, sorbitol and sodium benzoate as an oral source of creatine for an animal.

JP2004123686 describes a method for preventing heat stroke comprising administering a mixture of glycerol, water and raw royal jelly to a subject.

US5236712 describes compositions comprising water, electrolytes, alanine and glycerol, and use of such compositions for ameliorating the effects of physical exertion.

Greenwood et al. (2003) Molecular and Cellular Biochemistry, Vol. 244, No. 1-2, pages 83-88, describes a study of creatine supplementation by college football athletes.

Creatine (Cr) supplementation has been extensively used as a means of improving performance during short duration, high intensity exercise (e.g. Casey *et al*. 1996; Kilduff *et al*. 2002; Kreider *et al*. 1998). However, oral creatine supplementation has recently been shown to reduce cardiovascular, and thermoregulatory responses during exercise in the heat (Kilduff et al. 2004). The authors attributed the effects to increased cellular hydration due to a creatine-induced increase in TBW, and more specifically, intracellular water (ICW). A number of other studies have now shown an increase in ICW following creatine supplementation (e.g. Francaux & Poortmans, 1999) although the exact mechanisms behind this effect are as yet unknown.

Thus there is a need for further compositions which assist in uptake and retention of water by the body.

### Summary of the invention

In a first aspect, the present invention provides a method of increasing the capacity of a subject to absorb ingested water and/or to retain body water, comprising administering creatine and glycerol to the subject.

In preferred embodiments the methods of the invention comprise the step of administering glycerol to the subject at least once per day for at least two days, preferably for at least five days, and more preferably for at least 10 days.

In order to provide optimum benefit without side-effects, the dose of glycerol may be calculated depending on the body weight of the subject. Each administration preferably provides a dose of between about 0.1 g and about 2 g glycerol per kg body Weight, preferably between about 0.3 g and about 1.0 g glycerol per kg body weight, e.g. about 0.5 g glycerol or about 1.0 g glycerol per kg body weight.

Alternatively, a predetermined standard quantity of glycerol may be administered, without reference to the particular body weight of the subject. For example, a batch of a particular composition may be prepared for administration to a number of subjects. Any adverse reaction (e.g. onset of headaches etc.) may be taken into account individually if required, e.g. by adjusting the quantity of the composition administered to that subject, or by preparing a bespoke composition for them.

A standard quantity may be calculated for a typical 70 kg adult. Thus, a single administration may provide a dose of between about 7 g and about 140 g glycerol, preferably between about 21 g and about 70 g, e.g. about 37.5 g glycerol or about 70 g glycerol.

Typically, up to about 4 g of glycerol per kg body weight may be administered to a given subject per day. For example, a subject may receive between about 0.1 g and about 4 g per kg body weight per day, between about 0.3 g and about 3 g per kg per day, or between about 0.5 g and about 2 g per kg per day.

Thus a subject may receive up to about 280 g of glycerol per day. For example, a subject may receive between about 7 g and about 280 g glycerol per day, between about 21 g and about 210 g glycerol per day, or between about 37.5 g and about 140 g glycerol per day.

The total daily amount of glycerol may be provided in one, two, three, four or more doses, which may be separated by one, two, three, four or more hours. Preferably doses are separated by at least 4 hours. It will be appreciated that other dosing regimes are possible.

The methods of the invention may involve administration of a given daily quantity of glycerol (e.g. as described above) for a first period of time, in order to build up the amount of glycerol in the body. Subsequently, a smaller daily quantity of glycerol may be administered for a second period of time, in order to maintain the body's glycerol level. These first and second periods of time may be referred to as a "conditioning" period and a "maintenance" period respectively.

The conditioning period may, for example, be from one to fourteen days in length, preferably from three to ten days, preferably from five to seven days.

The maintenance period may be of indefinite duration, and preferably continues for as long as the body is exposed to high temperature conditions, or conditions which require intake of water to be controlled or minimised.

During the conditioning period, glycerol is typically administered two or more times per day, e.g. three, four or five times per day, as described above. During the maintenance period, glycerol is typically administered only once per day as described above, although administration may be more frequent if desired.

Preferably each individual dose administered during the conditioning and maintenance periods provides the same quantity of glycerol. The total quantity of glycerol administered daily during the conditioning period is typically between about two and about five times greater than the quantity administered during the maintenance period, e.g. three or four times greater.

By way of example only, a subject may receive about 2 g glycerol per kg body weight per day for the conditioning period, followed by about 0.5 g per kg per day during the maintenance period.

Thus a subject may receive about 140 g glycerol per day for the conditioning period, followed by about 37.5 g glycerol per day during the maintenance period.

Glycerol is preferably administered in aqueous solution.

The methods of the invention may involve administering creatine to the subject at least once per day for at least two days, preferably for at least five days, and more preferably for at least 10 days.

Each individual administration may comprise a dose of at least 0.5 g, at least 3 g, at least 5 g, or at least 10 g of creatine. However it is considered unlikely that the typical adult will be able to absorb much more than about 10g of creatine through the gut wall from a single oral dose. Thus an individual dose will not normally exceed 15 g of creatine and may provide about 5 g or about 10 g of creatine.
Typically between about 3 g and about 30 g of creatine will be administered per day, preferably between about 5 g and about 20 g per day.

Any suitable dosage pattern may be used. Where more than 10g creatine is given daily, it is preferable to administer the total daily quantity in two or more individual doses, each of no more than about 10 g. The doses may be separated by one, two, three, four or more hours, preferably about 4 hours, e.g. in order to maximise absorption from the alimentary canal.

As described above in relation to glycerol, a given daily quantity of creatine (e.g. as described above) may be administered for a first period of time (a "conditioning" period), in order to build up the amount of creatine in the body. Subsequently, a lower daily quantity of creatine may be administered for a second period of time (a "maintenance" period), in order to maintain the body's creatine level.

The conditioning period may, for example, be from one to fourteen days in length, preferably from three to ten days, preferably from five to seven days. The maintenance period may be of indefinite duration, and preferably continues for as long as the body is exposed to high temperature conditions.

During the conditioning period, creatine is typically administered two or more times per day, e.g. three, four or five times per day, as described above. During the maintenance period, creatine is typically administered only once per day as described above, although administration may be more frequent if desired.

Preferably each individual dose administered during the conditioning and maintenance periods provides the same quantity of creatine. The total quantity of creatine administered daily during the conditioning period is typically between about two and about five times greater than the quantity administered during the maintenance period, e.g. three or four times greater.

Preferably about 20 g of creatine is administered per day during the conditioning period and about 5 g of creatine is administered per day thereafter.

The creatine is preferably administered in aqueous solution. Solutions may be prepared from any suitable form of creatine, for example creatine monohydrate or creatine phosphate.

Preferably the method comprises administering carbohydrate to the subject before or with the creatine. Without wishing to be bound by any particular theory, it is believed that the carbohydrate will stimulate insulin secretion, which will aid the uptake of creatine from the gut.

Where the carbohydrate is administered before the creatine, it is preferably given not more than about three hours before the creatine. Preferably it is given between one and three hours before the creatine, depending on the type and complexity of the carbohydrate, in order to provide an optimum insulin response. However acceptable levels of creatine uptake are achieved when the carbohydrate is given simultaneously with the creatine.

The carbohydrate preferably comprises a polysaccharide, which in preferred embodiments is glucose polymer. The chain length of the polymer may be chosen depending on the desired osmolality of the final composition containing it (see below); the longer the chain length, the lower the osmolality will be. Maltodextrin, comprising seven glucose units, is an example of a suitable polymer.

A single dose preferably provides at least about 30 g of glucose, e.g. about 70 g.

The amount of carbohydrate may be calculated based on the amount of creatine present in the composition. Carbohydrate may, for example, be present at about 3 to 10 parts by weight per part by weight of creatine, e.g. about 7 parts by weight per part creatine. Alternatively the carbohydrate dosage may be calculated depending on the recipient's body weight, in which case at least about 0.4 g should be given per kg body weight, e.g. about 1g per kg.

Periods taking creatine and/or glycerol alone may be alternated with periods taking both creatine and glycerol.

Creatine and glycerol may be administered simultaneously or at different times, and in the same or different compositions. Preferably the creatine and glycerol are administered in a single composition, which may also contain any carbohydrate to be administered.

The composition is preferably an aqueous solution. Compositions of the invention are typically suitable for oral administration, e.g. as a drink. However, other modes of administration, e.g. intravenous administration, may also be used.

The methods of the invention typically further comprise the administration of water to the subject. The glycerol, creatine and carbohydrate (if any) will normally be dissolved in water, and further water will normally be ingested between doses of creatine and glycerol. Conventional rehydration regimes may be used in conjunction with the methods and compositions of the invention. These may involve ingestion of water and electrolyte supplements, such as conventional sports drinks.

The methods of the invention may be used in non-therapeutic contexts, for example to improve athletic performance at high ambient temperatures. Both physical and mental performance may be improved; it is well-established that an increase in core temperature causes mental fatigue as well as having effects on physical performance.

"Water loading" by administration of creatine and glycerol as described herein causes a large increase in total body water, and also significantly reduces the increase in heart rate and core body temperature caused by strenuous exercise, particularly in the heat. In order to achieve these benefits during a particular session of strenuous exercise (e.g. marathon running, cycling, etc.), it is preferred that a final administration of glycerol and/or creatine is given not less than about 5 hours before that session of exercise. If less time elapses between administration and exercise, the full hydration benefits may not be achieved. This may be because the glycerol and/or creatine are only absorbed slowly from the stomach into the tissues, and so are not able to exert their full osmotic effects on intracellular and extracellular water until about 5 hours after administration. However absorption may occur differently in different individuals, so the ideal timing of administration may vary from subject to subject.

Water may of course be administered less than 5 hours after final creatine/glycerol administration. In fact, this is normally desirable, in order to ensure that the subject is fully hydrated before beginning exercise. Thus, at least about 500 ml of water is preferably administered about 3 hours and/or about 1 hour before exercise.

The conditioning program may resume after exercise, for example to prepare the body for a subsequent session of exercise.

The timing of creatine/glycerol administration is not so critical when a subject is being conditioned for normal day-to-day activity or need only undertake moderate exercise.

The methods of the invention are not restricted to use in high-temperature environments, or to providing benefits during strenuous exercise. They may be applied in any situation in which excessive water loss (e.g. by sweating) may cause difficulty, and/or in which it is desirable to control or minimise intake of water (e.g. when availability of water is restricted). The methods increase the proportion of ingested water retained in the tissues, and reduce that lost to urine. They also reduce the amount of water lost to sweat under high temperature conditions and/or during physical exertion. Thus the methods may be useful for anyone exposed to high temperature conditions, especially where water is not freely available, such as military personnel on desert operations. The body's need for water is believed to increase with altitude, so mountaineers may find the methods of the invention useful in order to reduce the amount of water they must carry on an expedition. Similarly, if the methods of the invention were applied to astronauts, the amount of water required in the payload of a spacecraft could be reduced. Other applications will be apparent to the reader.

The compositions and medicaments may be used therapeutically, in the prevention or treatment of dehydration and/or heat stroke, heat cramps and heat exhaustion. Heat stroke is considered to occur when the body temperature reaches 41°C or above. Supplementation of creatine and glycerol levels by the methods of the invention improves water absorption and retention, with concomitant benefits on thermoregulation, providing significant reduction in core temperature increase under high temperature conditions. Thus the methods of the invention may attenuate the increase in core temperature which would otherwise give rise to heatstroke.

Thus the invention further provides the use of creatine in the manufacture of a medicament or composition for the prevention or treatment of dehydration, heat stroke, or heat-related illness as defined in the claims, wherein the medicament or composition is for administration in conjunction with glycerol.

The invention further provides the use of glycerol in the manufacture of a medicament or composition for the prevention or treatment of dehydration, heat stroke, or heat-related illness as defined in the claims, wherein the medicament or composition is for administration in conjunction with creatine.

The invention further provides the use of creatine and glycerol in the manufacture of a medicament or composition for the prevention or treatment of dehydration, heat stroke, or heat-related illness, as defined in the claims.

The medicaments or compositions of the invention may further comprise carbohydrate, preferably a polysaccharide, more preferably glucose polymer.

The invention further provides creatine and glycerol for use in a method of medical treatment, optionally in conjunction with carbohydrate.

In yet further aspects, the invention provides compositions for increasing the capacity of a subject to absorb and/or retain ingested water, and medicaments for the prevention and/or treatment of dehydration and other heat-related illnesses. The compositions of the invention include ready-to-drink solutions as well solid and liquid concentrates to be dissolved, suspended or diluted in an appropriate manner, most preferably in water. Compositions suitable for administration by other methods, e.g. intravenously, are also provided.

The compositions of the invention comprise creatine and glycerol. Typically, the compositions comprise at least about 4 parts by weight of glycerol per part by weight of creatine. Preferably the compositions have up to about 20 parts by weight of glycerol per part by weight of creatine, although higher ratios of glycerol to creatine are possible.

In preferred embodiments the compositions comprise up to about 20 parts glycerol per part creatine, and preferably at least about 5 parts glycerol per part creatine. For example, the composition may comprise about 6, 7, 8, 9, 10, 11, 12, 13, 14, 15, 16, 17, 18 or 19 parts glycerol per part creatine. A preferred composition comprises 5 to 10, e.g. 7, parts glycerol per part creatine. By way of illustration, the embodiment described in the Example below contains approximately 7.2 parts glycerol per part creatine.

The compositions of the invention preferably also comprise carbohydrate, preferably a polysaccharide, preferably glucose polymer.

A liquid composition may contain at least about 30 g of carbohydrate per half litre.

Preferably, about 5 to 10 parts by weight carbohydrate are present per part by weight of creatine, e.g. about 7 parts by weight carbohydrate per part creatine.

An exemplary composition, suitable for administration to a typical 70 kg adult, might contain between about 5 g and 15 g creatine, e.g. about 10 g, per litre. It may contain between about 60 g and 80 g glycerol, e.g. about 70 g, per litre. Carbohydrate may be added as appropriate, e.g. to between 30 and 100 g glucose per litre, e.g. to about 75 g glucose per litre.

The compositions of the invention may comprise additional ingredients, such as electrolytes.

The compositions may be provided as ready-to-drink solutions, or as solid or liquid concentrates, which may be dissolved or diluted before administration, preferably in water.

The compositions may be provided in kit form, optionally with creatine and glycerol present in separate containers. Such kits may comprise instructions for preparing a composition of the invention and/or for administration according to the methods of the invention as set out above.

### Description of the Drawings

Figure 1 shows a schematic representation of the experimental design.
Figure 2 shows a schematic representation of the experimental protocol.
Figure 3 shows changes in body mass (BM), total body water (TBW), intracellular water (ICW) and extracellular water (ECW) after combined Cr and Gly supplementation with final supplement consumed 3 hrs (A) and 5 hrs (B) prior to measurement.
Figure 4 shows changes in body mass (BM), total body water (TBW), intracellular water (ICW) and extracellular water (ECW) in the creatine-supplemented and placebo groups. Data is presented as the mean ± sd. †: indicates a significant greater change.
Figure 5 shows oxygen uptake during exercise before and after supplementation in the two groups. Data presented as the mean ± sd. No significant differences were found between trials in either group.
Figure 6 shows heart rate during exercise before and after supplementation in the two groups. Data presented as the mean ± sd. †: indicates a significant difference pre- vs post-supplementation.
Figure 7 shows RPE for perceived leg fatigue during exercise before and after supplementation in the two groups. Data presented as the mean ± sd. †: indicates a significant difference pre- vs post-supplementation.
Figure 8 shows RPE for breathlessness during exercise before and after supplementation in the two groups. Data presented as the mean ± sd. †: indicates a significant difference pre- vs post-supplementation.
Figure 9 shows rectal temperature during exercise before and after supplementation in the two groups. Data presented as the mean ± sd. †: indicates a significant difference pre- vs post-supplementation.
Figure 10 shows changes in plasma volume during exercise before and after supplementation in the two groups. Data presented as the mean ± sd. †: indicates a significant difference pre- vs post-supplementation.
Figure 11 shows time trial performance before and after supplementation in the two groups. Data presented as the mean ± sd. No significant differences were found between trials in either group.

### Detailed Description of the Invention

The present invention relates to methods and compositions for increasing the capacity of a subject to absorb and/or retain ingested water, as well as for the prevention and/or treatment of dehydration and other heat-related illnesses.

Without wishing to be bound by any particular theory, the compositions and methods of the invention are believed to be particularly effective because they provide a combination of components which together promote uptake and retention of water by both the intracellular and extracellular fluid spaces.

The inclusion of glycerol, a three carbon alcohol synthesised naturally in the body, is aimed primarily at enhancing extracellular water retention. The glycerol exerts an osmotic effect but only in the extracellular space as glycerol cannot be taken up by skeletal muscle. Reports in the literature about the hydrating effect of glycerol are inconclusive, with some alleging increased hydration and performance, and others apparently finding no effect.

Glycerol is only slowly eliminated by the liver and so will tend to accumulate in the body if administered sufficiently regularly and frequently. As a result, there is no particular minimum dose which must be given. Individual doses of glycerol above about 1 to 1.5 ml (approx. 1.26 to 1.89 g) per kg body weight tend to produce side-effects, such as headaches. Each single dose of glycerol is ideally kept at or below these levels. A dose of approximately 0.375 to 0.75 ml (approx. 0.5 g to 1 g) per kg body weight is preferred. For a dose of this magnitude, doses are preferably separated by at least 4 hours in order to avoid side effects. Preferably, not more than 4 g glycerol will be given per kg body weight per day. However it will be understood that tolerance to glycerol and its side-effects is variable between individuals, so dosages may be tailored according to the requirements of any particular subject.

Creatine (methyl guanidine acetic acid) is synthesised naturally in the body. A typical 70 kg adult has approximately 120 g of creatine in the body, of which 95 % is located in skeletal muscle.

The role of creatine in the compositions and methods described herein is to increase intracellular water retention. Added benefits include the "anabolic" and "ergogenic" properties of creatine. It also provides quickly available energy.

It has previously been established that creatine supplementation results in increased intracellular water and total body water, although the mechanism behind this remains unclear. However the hydration associated with creatine uptake by muscle has generally been regarded as an undesirable side-effect of creatine administration. Creatine is most frequently used to increase muscle mass in the field of body-building, where the associated influx of water is generally unwelcome because it tends to reduce muscular definition.

Creatine has not been associated with any physiologically harmful side-effects. It tends to accumulate in the muscles, being washed out from there only very slowly. There are therefore no particular bounds within which the dose of creatine must be kept for safety.

Creatine is known to enhance characteristics including muscle mass, strength, endurance performance, and performance in repeated high-intensity exercise. The methods and compositions of the present invention will therefore provide these benefits in addition to hyperhydration.

It is thought unlikely that the adult human body can absorb much more than about 10 g of creatine in a single oral dose; a single dose is therefore unlikely to exceed 20 g for a normal 70 kg adult.

The creatine will typically be administered to the subject in aqueous solution. Compositions comprising creatine may be made up using any appropriate form of creatine, including crystalline creatine (e.g. creatine monohydrate) or creatine salts (e.g. creatine phosphate). However references in this specification to parts by weight of creatine should be taken to mean parts by weight of creatine as such, i.e. methyl guanidine acetic acid, having a relative molecular mass of 131.12.

The present inventor has realised that the hydrating properties of creatine can be harnessed to improve water uptake, water retention and thermoregulation, especially when used in conjunction with an agent which promotes hydration of the extracellular space.

In the methods of the invention, creatine is thought to exert an osmotic effect from within the cells, to increase intracellular water. However this would be expected to reduce extracellular water, possibly with deleterious effects. Glycerol exerts an osmotic effect in the extracellular space to increase extracellular water. However, this would also be expected to have deleterious effects because it will tend to reduce intracellular water. The methods and compositions of the present invention have been found to provide the benefits associated with the hydrating properties of both creatine and glycerol, but each appears to compensate for the deficiencies of the other. Thus a combination of creatine and glycerol provides beneficial effects which are greater than the sum of those provided by the two components alone. This could not have been predicted.

Preferably carbohydrate is administered either before or with the creatine. The carbohydrate acts to increase body insulin levels, which in turn increases creatine uptake. Without carbohydrate, creatine uptake may occur at sub-optimal levels.

In addition to stimulating creatine uptake, the carbohydrate may also be used to provide a source of readily and rapidly available fuel.

The carbohydrate is preferably administered as a polysaccharide, especially when administered in the same composition as the creatine and glycerol. This is to keep the osmolality of the composition as low as possible. Nevertheless, the osmolality of combined creatine/glycerol/carbohydrate compositions as described in this specification may still be high enough to provoke mild gastrointestinal side-effects in some individuals. The polysaccharide is preferably glucose polymer. Suitable flavourings may also be provided.

The carbohydrate is preferably administered at about 5 to 10 parts by weight per part by weight of creatine.

In order to achieve a suitable accumulation of creatine and glycerol in the body, it is recommended that glycerol and creatine are each administered to the subject at least once per day for a period of a number of days, i.e. at least two days, preferably for at least five days, and more preferably for at least 10 days. Administration may be once per day, or more frequently if desired, for example two, three, four or five administrations of each component, either together or separately.

Particularly beneficial effects are obtained if the glycerol and/or the creatine are each administered at least twice, and preferably three or four times per day for a first "conditioning" period in order to achieve a rapid build-up of the creatine and glycerol within the body and provide maximal beneficial effects in a short time. These beneficial effects can thereafter be maintained by less frequent administration of each component for a second "maintenance" period; once per day may be sufficient after a five to ten day first period, although administration during the second period may be more frequent if required.

The conditioning period may, for example, be from one to fourteen days in length, preferably from three to ten days, preferably from five to seven days. The maintenance period preferably lasts for as long as the body is exposed to high ambient temperatures.

It may be desirable to condition the body to increase its absorption of ingested water and/or retention of body water before a period of exposure to high temperatures. This may be achieved by subjecting the body to a conditioning period of administration of creatine and glycerol before exposure to a high temperature environment. Such advance preparation will minimise the amount of fluid which needs to be ingested once the body is exposed to the high temperature environment.

For example, a regime according to the invention may involve administration of about 2 g of glycerol per kg body weight per day for seven days, followed by a single dose of about 1 g per kg per day. The 2 g total in the conditioning period may be split into two, three or four doses as required.

Administration of creatine and glycerol according to the methods described herein can have two separate but related effects on the body.

Firstly, it will increase the body's capacity to absorb ingested water into the tissues from the gastrointestinal tract. That is to say, conditioning of the body by accumulation of creatine and glycerol enables the body to absorb a higher proportion of water from ingested drinks than would have been the case without such conditioning. To put it another way, conditioning by the methods of the invention increases the proportion of ingested water absorbed into (and retained in) the tissues, and reduces the proportion lost to urine.

Secondly, it increases the body's capacity to retain water during physical activity and/or under high ambient temperature conditions, so reducing the amount of water lost to sweat etc.. The improvement in water absorption and retention will be accompanied by improvements in thermoregulation, which will tend to attenuate increases in body core temperature and heart rate which occur when exposed to high temperatures. This may be useful to avoid heat stroke, as described above, or to decrease the perception of effort during exercise. The methods and compositions of the invention show particular benefits in increasing total body water, and attenuating the rise in heart rate and core body temperature caused by strenuous exercise under high temperature conditions.

It will be appreciated that compositions containing creatine and glycerol as described herein are not themselves intended to supply the fluid necessary to hydrate or rehydrate a subject. These compositions normally contain such a high level of dissolved solutes that they are hypertonic as compared to body fluids, which is not conducive to absorption of water from the GI tract.

Rather, the methods and compositions described are intended to be used in conjunction with other means of hydration. Subjects undergoing a conditioning regime as described will typically take on additional water (optionally also containing electrolytes, sugars, etc.) in order to become or remain suitably hydrated. Thus the methods and compositions of the invention may be used in conjunction with any conventional hydration protocols.

The invention also provides ready-to-drink solutions, as well as concentrates for dissolving, suspending or diluting into drinkable compositions. Such compositions typically contain all the components necessary to put the invention into practice.

Also provided are compositions for other modes of administration, such as intravenous administration. For intravenous administration the composition will preferably be in the form of a parenterally acceptable aqueous solution which is pyrogen-free and has suitable pH, tonicity and stability. Those of relevant skill in the art are well able to prepare suitable solutions. Preservatives, stabilisers, buffers, antioxidants and/or other additives may be included, as required.

Thus a suitable aqueous composition will contain glycerol, creatine and normally a form of carbohydrate, typically glucose in a monomeric or polymeric form, or a mixture of forms. Additional components such as electrolytes (e.g. sodium, potassium and chloride) may also be present if desired.

The precise dose of each component in a particular composition may be tailored (as described above) according to the requirements of the subject to whom the composition is to be given. It will be appreciated that the volume of any particular composition is less important than the dose of each component to be administered and the relative amounts of each component present. For ease of oral consumption, volumes of 0.5 to 1 litre per administration are preferred.

The osmolality of the composition is also significant. Compositions with very high osmolality may cause gastrointestinal irritation and are generally less palatable than compositions having lower osmolality. Preferably the osmolality is kept below about 1200 mosmol/kg. A value of between 900 to 1100 mosmol/kg has been found to be generally acceptable.

A composition comprising 53 g of glycerol, 10 g of creatine and 75 g of glucose polymer can be readily prepared in a volume of 1 litre within this range of osmolality. For example, such a composition may be prepared by dissolving 75 g glucose polymer, 11.4 g creatine monohydrate and 53 g glycerol per litre final volume, in hot to boiling water.

One litre of this composition provides approximately 10 g of creatine and approx. 0.75ml/kg glycerol for a 70 kg adult (i.e. approx. 1g/kg body mass, as 0.75 ml of glycerol weighs 1.03 g). Two litres of the composition may be drunk per day for a period of seven days and half a litre per day thereafter. During the initial period, the two litre quantity may be administered as two doses of 1 litre each, as four doses of 0.5 litres each, or differently if required. Doses are preferably separated by 4 hours or more to avoid side-effects induced by glycerol.

It is recommended that the subject also consumes at least 1 litre of water per day, and preferably two litres or more, in addition to this composition to achieve optimum hydration.

Throughout this specification, use of the term "about" in conjunction with a numerical value is intended to encompass a possible deviation of up to 10% from the specified value, more preferably 5%, 4%, 3%, 2% or 1%.

### Examples

### Methods

*Subjects.* 18 well-trained cyclists gave their written informed consent to take part in the present study which was approved by the local Ethics Committee (Table 1). Subjects were recruited from local cycling clubs, and none were acclimatised to exercise in the heat at the time of study. Subjects were interviewed to ensure all were creatine-free for at least 8 weeks prior to the study. The investigators did not reveal prior to the interview that subjects would be excluded if they had supplemented with Cr in the previous 8 weeks. The subjects were fully informed of any risks and discomforts associated with the experiments before giving their written informed consent to participate.

**Table 1: Physical characteristics of the two groups of subjects.**

| | **Placebo Group** (n=9) | **Creatine Group** (n=9) |
|---|---|---|
| Age (yr) | 33 ± 9 | 31 ± 6 |
| Height (cm) | 174 ± 4 | 177 ± 3 |
| Weight (kg) | 74.7 ± 7 | 75.2 ± 6 |
| V̇O_{2 max} (L·min⁻¹) | 4.1 ± 0.3 | 4.2 ± 0.4 |
| WRₘₐₓ (W) | 346 ± 20 | 362 ± 34 |

*Experimental design.* All subjects had their maximal oxygen uptake (V̇O_{2 max}), maximal work rate (WRₘₐₓ) and lactate threshold (LT) measured during an initial continuous incremental test to volitional exhaustion at standard room temperature (20-21°C). LT was estimated non-invasively as the V̇O₂ at which: (a) the break-point in the relationship between CO₂ production (V̇CO₂) and V̇O₂ ("V-slope" technique, Beaver et al. 1986) occurred and (b) the ventilatory equivalent for O₂ (V̇E/V̇O₂) started to increase systematically without a concomitant increase in ventilatory equivalent for CO₂ (V̇E/V̇CO₂). An electrically braked cycle ergometer (Excalibur Sport, Lode, The Netherlands) was set at an incrimination rate of 15 W·min⁻¹. Respired volumes were measured with a bi-directional turbine transducer (VMM; Alpha Technologies, Laguna Niguel, CA, U.S.A.), calibrated with a 3-litre syringe, using a range of different flow profiles (Hans Rudolph, Kansas City, MO, U.S.A.). Respired gas concentrations were measured every 20 ms by a quadrupole mass spectrometer (QP9000; Morgan Medical, Gillingham, Kent, U.K.), which was calibrated against precision-analysed gas mixtures. Barometric pressure was measured using a standard mercury barometer.

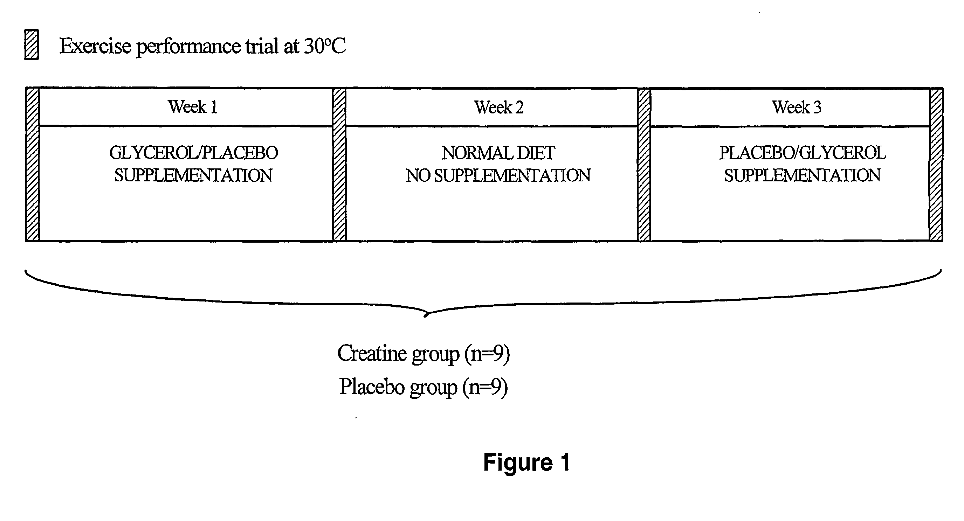

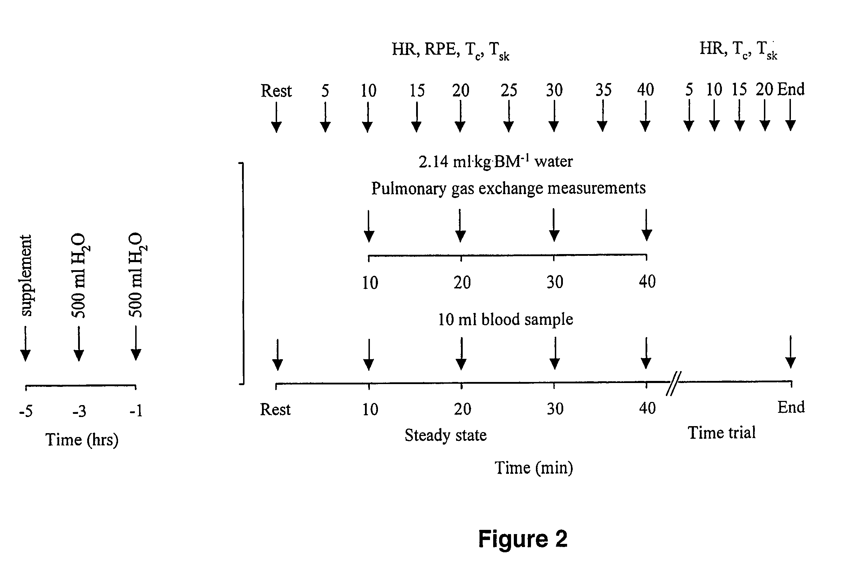

The experimental design and protocol are complicated and is best understood by reference to Figures 1 and 2. The study consisted of two supplementation regimens, each lasting 7 days and encompassing four cycle performance trials consisting of 40 min constant-load exercise at 63 % WRₘₐₓ followed by a 16.1 km (10 mile) time trial. Prior to the first of these experimental trials, familiarisation trials were completed until the variability of two consecutive trials was within 5%. Following this familiarisation period, subjects were matched for body mass (BM) and were randomised in a double blind fashion to either a Cr or a placebo (P1) group. Subjects performed an exercise trial pre- and post-supplementation in both supplementation weeks. The first test was conducted at least 48 hours after each subject's final familiarisation trial. Each supplementation period started on the day after the first test and finished on the day of the second test.

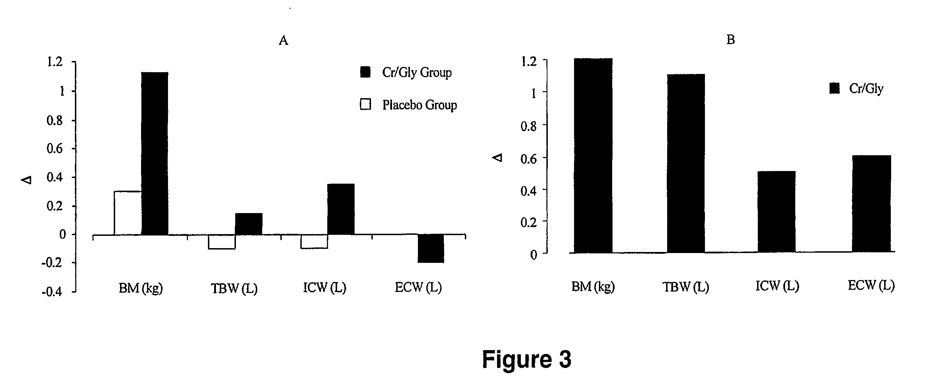

Cr supplementation consisted of 22.8 g·d⁻¹ of Cr·H₂O (equivalent to 10 g Cr x 2 daily) and 140 g of glucose polymer (70 g x 2 daily) made up in 2 L of warm to hot water for 6 days. This protocol has been shown to increase resting muscle phosphocreatine (PCr) levels within 5 days (Harris et *al.* 1992). Each supplementation was made freshly prior to consumption in order to prevent any degradation of Cr to creatinine (Crn). The P1 supplement consisted of 170 g·d⁻¹ of glucose polymer (85 g x 2 daily). During the first and third week of the experimental regimen, subjects in both groups received either 1 g glycerol (Gly) per ·kg BM or an equivalent amount of P1 (sucrose solution) diluted in each supplement. In both post-supplementation trials (days 8 and 22) subjects consumed the appropriate supplement 5 hours prior to exercise. Hypertonic solutions, such as the Cr/Gly combination (965 ± 61 mosmol·kg⁻¹) cause an initial net secretion of water into the intestinal lumen (Gisolfi et al. 1990) resulting in an effective loss of body water (see Figure 3A). Unpublished work in our lab has indicated that at least 5 hours must be left between consumption of the supplement and commencement of exercise in order to allow full absorption of all the water into the fluid compartments (Figure 3B). All supplements had similar taste, texture and appearance and were placed in generic water bottles to ensure double blind administration. On each of the experimental test days (i.e. days 1, 8, 15, 22) subjects ingested 500ml of water 3 hours prior to exercise and a further 500ml of water 1 hour prior to exercise to ensure subjects were adequately hydrated prior to all exercise trials (American College of Sports Medicine, 2000).

Subjects otherwise followed their normal diet and weighed all food and drink consumed during the supplementation period, using digital weighing scales, readable to 1 g. The diet was analysed for energy intake and macronutrient content using a computerized version of McCance and Widdowson's food composition tables as revised by Holland et *al.* (1991). Subjects were asked to minimise caffeine intake to lessen the possible inhibitory effects of caffeine on the ergogenic effect of Cr (Vandenbeghe et *al.* 1996). Subjects completed 7 separate 24 hr urine collections. The collection began on the day preceding supplementation (baseline), then continued through the 6 days of supplementation. The urine volume for each 24 hr period was measured and mixed thoroughly, with a representative 20 ml sample being stored at -20°C for subsequent analysis (ABX Mira Plus Spectrophotometer, ABX diagnostics, UK) of [Cr] and [Crn].

Procedures. Subjects reported to the laboratory on the day of testing following a 3 hr fast and having refrained from alcohol, caffeine and strenuous exercise the day before. Upon arrival at the laboratory, height and nude BM were measured and body water compartments were estimated using a Bodystat Multiscan 5000 Bioimpedance analyser (Bodystat Ltd, Isle of Man) (Van Loan, 1990). This method allows TBW and ECW to be estimated; from these measurements ICW can also be deduced. The bioimpedance measurements were taken while the subjects lay comfortably in a supine position on a non-conductive surface with their arms and legs slightly abducted. Following the bioimpedance measurement, a flexible rectal thermistor was inserted 10 cm beyond the anal sphincter to measure rectal temperature (Tᵣₑ), an index of core temperature (T_{c}) and a heart rate (HR) monitor (Polar Sports Tester, Polar Electro Oy, Kempele, Finland) was attached. The subject's right hand and forearm were immersed in water at 42-44°C for 15 min in order to allow for arterialisation of the venous blood (Forster at al. 1972). Following this, a 21G cannula was introduced into a superficial vein on the dorsal surface of the heated hand. The subject was transferred to the climatic chamber (ambient temperature 30 ± 1°C with a relative humidity of 70 ± 2% and air velocity of approximately 1.8 m·sec⁻¹) and seated on the cycle ergometer for 5 min. During this period thermistors (C8600 10 channel microprocessor, Comark, Hertfordshire, UK) were attached to the subject's chest, tricep, thigh and calf for the determination of weighted mean skin temperature (Tₛₖᵢₙ) (Ramanthan, 1964). The subject remained seated on the cycle ergometer for a further 1 min while resting HR, Tᵣₑ, Tₛₖᵢₙ were recorded and a blood sample (10 ml) obtained. The venous cannula was kept patent by a 10 ml infusion of isotonic saline between samples. Subjects were then instructed to begin 5 min of unloaded cycling before the WR was increased in a "single step" to the predetermined 63% WRₘₐₓ. Subjects were required to maintain a pedal cadence of 70-100 rpm for 40 min. Measurements of HR, Tᵣₑ and Tₛₖᵢₙ were recorded at 5 min intervals throughout the 40 min period and during the time trial. Blood samples (10 ml) were obtained every 10 min during the steady state exercise and upon completion of the time trial. One minute expired gas collections were made every 10 min during steady state exercise and analysed within 5 min for the determination of V̇O₂, V̇CO₂, V̇E and respiratory exchange ratio (RER). Subjects were required to consume 2.14 ml · kg · BM⁻¹ of water every 10 min throughout the 40 min steady state period. Ratings of perceived exertion (RPE) for leg fatigue and dyspnoea were recorded every 5 min of the steady state exercise and at the end of the time trial using the Borg category scale (Borg, 1982). On completion of the 40 min period the WR was decreased to 20 W and the subject asked to maintain cadence for 1 min. After a further 4 min rest period the subject was instructed to complete a 16.1 km (10 mile) self-paced time trial on a road mounted cycle (King Cycle Indoor Trainer, Buckinghamshire, UK). After exercise, nude BM was measured and the difference in BM before and after exercise was calculated and subsequently used to estimate sweat rate and sweat loss, after correcting for respiratory water loss and substrate oxidation (Mitchell et al. 1972). The time trial completion time was recorded but withheld from the subject until all exercise tests had been completed.

*Blood treatment and analysis.* Blood was drawn into dry syringes and 6 ml dispensed into a tube containing K₃EDTA and the remaining 4 ml dispensed into a tube without K₃EDTA. Duplicate aliquots (400µl) of whole blood from the K₃EDTA tube was rapidly deproteinised in 800 µL of ice cold 0.3 N perchloric acid, centrifuged and the supernatant used for the measurement of glucose, pyruvate, and lactate using standard enzymatic methods with spectrophotometric detection (Mira Plus, ABX Diagnostics, Montpellier, France). A further aliquot of blood was centrifuged and the plasma obtained was separated and used for the measurement of FFA (colorimetric method, Roche Diagnostics GmbH, Germany) and glycerol (Boobis & Maughan, 1983). The blood in tubes without anticoagulant was allowed to coagulate and then centrifuged; the serum collected was used for the measurement of osmolality by freezing point depression (Micro-osmometer 3300, Vitech Scientific, West Sussex, UK) The blood from the K₃EDTA tubes was also analysed for haemoglobin (Hb) (cyanmethaemoglobin method, Sigma, Chemical Company Ltd., Dorset, UK) and packed cell volume (PCV) (conventional microhematocrit (Hct) method). All blood analyses were carried out in duplicate with the exception of PCV, which was carried out in triplicate. PV changes were calculated from changes in Hb and PCV relative to initial baseline values (Dill & Costill, 1974).

*Calculations.* Weighted mean skin temperature [Tₛₖ=0.3 (T_{chest}+T_{tricep}) + 0.2 (T_{thigh}+T_{calf}) ] (Ramanthan, 1964) and mean body temperature (T_{b}) [0.87Tᵣₑ+0.13Tₛₖ] (Olschewski & Bruck, 1988) were calculated for each time point. Metabolic rate was calculated for each time point using the following equation: metabolic rate= [4.686+ (RER-0.707/0.293)0.361] V̇O₂ (Ravussin et *al.* 1985).

*Data analysis* Data were expressed as the mean ± s.d. or median (range), following a test for the normality of distribution. Statistical analysis was carried out using three factor mixed model ANOVA, followed by a simple main effects analysis for significant three way interactions (i.e. pre- vs. post-supplementation at each combination of time point and treatment) and simple main effects analysis for two way interactions. In addition, the magnitude of change (Δ) in the four supplement regimens (P1/P1, P1/Gly, Cr/Pl and Cr/Gly) were examined using two-sample t-test when significance was identified using the simple main effects analysis. An ANCOVA was used where necessary to normalise for differences in pre-supplementation results using the baseline value as the covariate. Statistical significance was declared at P≤0.05.

### Results

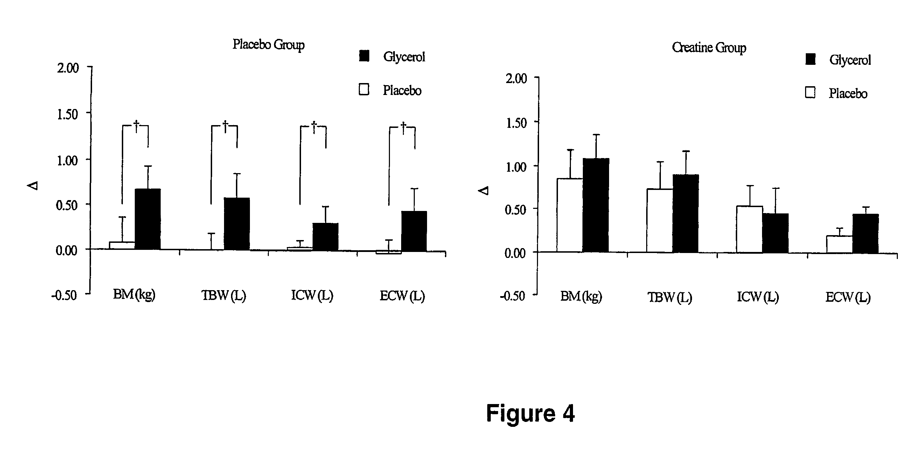

*Body mass and water compartments.* The physical characteristics of the two groups were similar before supplementation (Table 1). In the P1 group, BM increased significantly following Gly supplementation, with no change during the placebo week (Δ BM was greater in the Gly supplementation week, Figure 4). In the Cr group, BM increased significantly during both the placebo week and Gly week. Furthermore, there was a tendency for the increase in BM to be greater when Gly was consumed in combination with Cr than when Cr was consumed alone (P=0.051) (Figure 4). There was no difference pre-supplementation in TBW, ICW and ECW between groups. In the P1 group, TBW and ECW increased significantly following Gly supplementation, whereas TBW and ECW were unaltered in the P1 week. There was no significant increase in ICW in either supplementation week in the P1 group. In the Cr group, TBW, ICW and ECW increased significantly in both supplementation weeks. Additionally, in the Cr group the increase in TBW tended to be greater in the Gly supplementation week than the P1 week (P=0.070) (Figure 4).

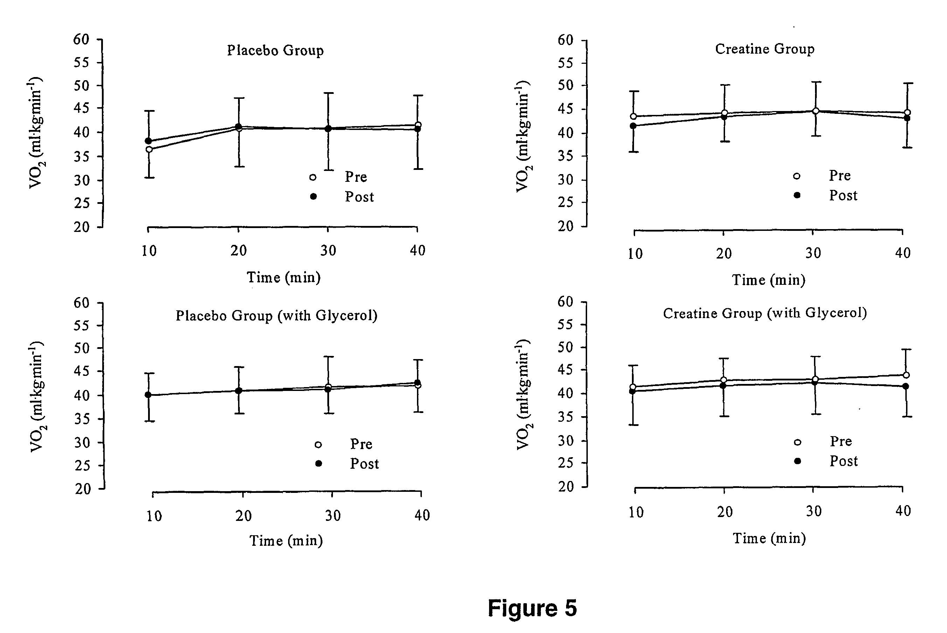

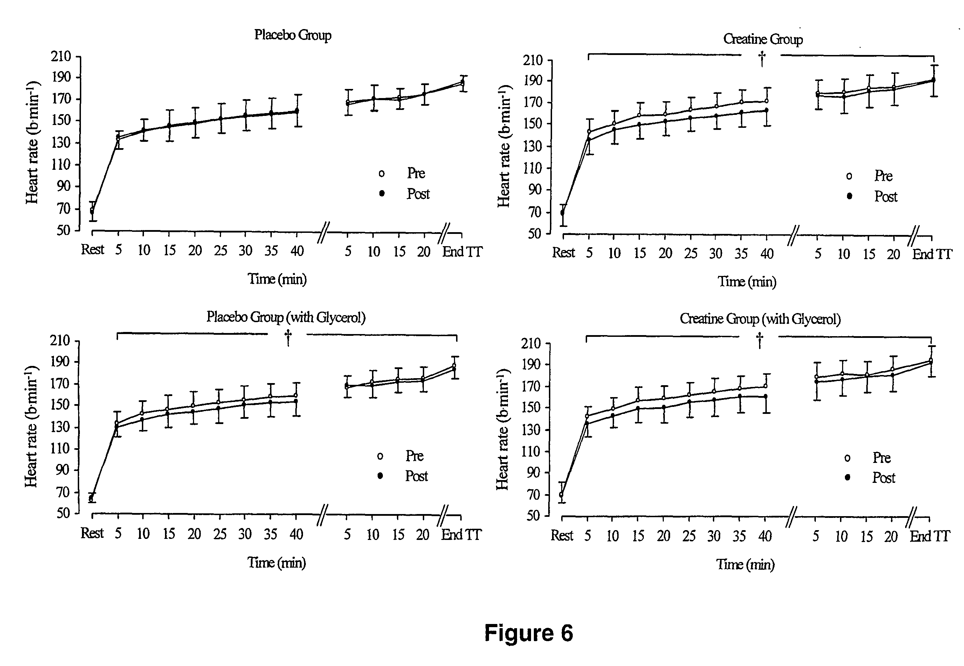

*Cardiopulmonary variables.* V̇O₂, V̇CO₂ and V̇E remained constant during steady state exercise and no differences were found between groups or as a result of supplementation (e.g. Figure 5). There were no differences in resting HR between supplementation trials (Figures 6). During exercise, HR increased during all trials. A three way interaction (pre-post x group x Gly) was examined through a simple main effects analysis and revealed that in the P1 group, HR during exercise was significantly lower following Gly supplementation compared to pre-supplementation (P=0.008) (Figure 6). In the Cr group, HR was significantly lower in both P1 and Gly supplementation weeks compared to pre-supplementation (Figure 6). Furthermore, the Δ HR between pre and post supplementation was significantly greater during the Gly supplementation week (P=0.017) .

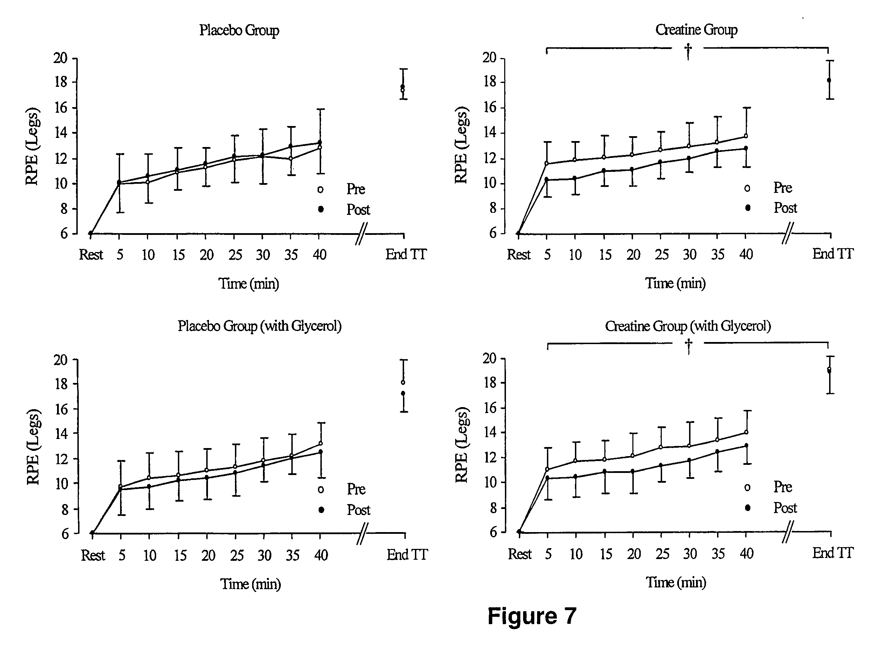

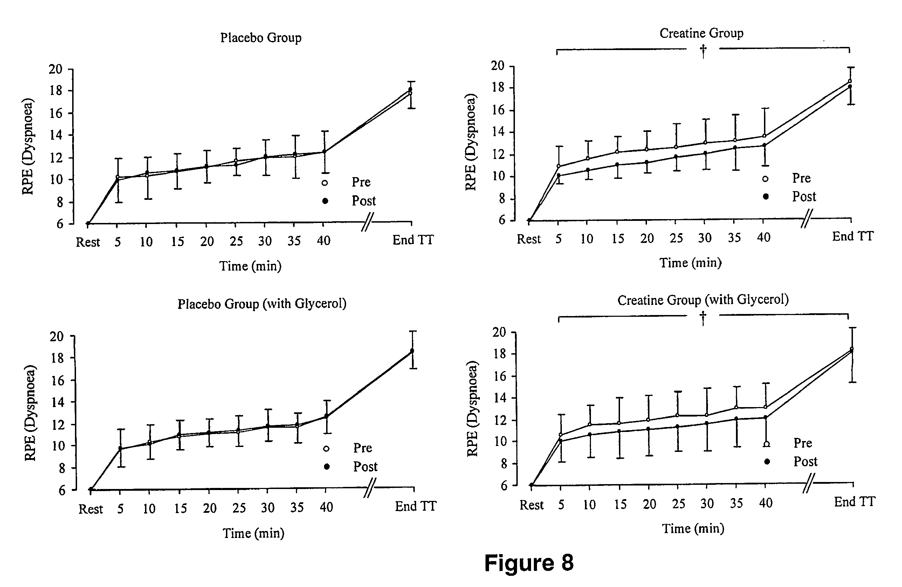

*Ratings of Perceived Exertion.* There was a progressive rise in RPE both for perceived leg fatigue and breathlessness during exercise reaching near maximum ratings at the end of the time trial (Figures 7 and 8). A significant three way interaction (P=0.007) was observed in RPE for perceived leg fatigue (Figure 7). Significantly lower ratings of perceived leg fatigue were found in the Cr group in both supplementation weeks; no such effect was found in the P1 group in either supplementation week (Figure 7). There was also a significant three way interaction with regard to RPE for breathlessness (P=0.015). Lower ratings of breathlessness were also found in the Cr group in both supplementation weeks; no such effect was found in the P1 group in either supplementation week (Figure 8).

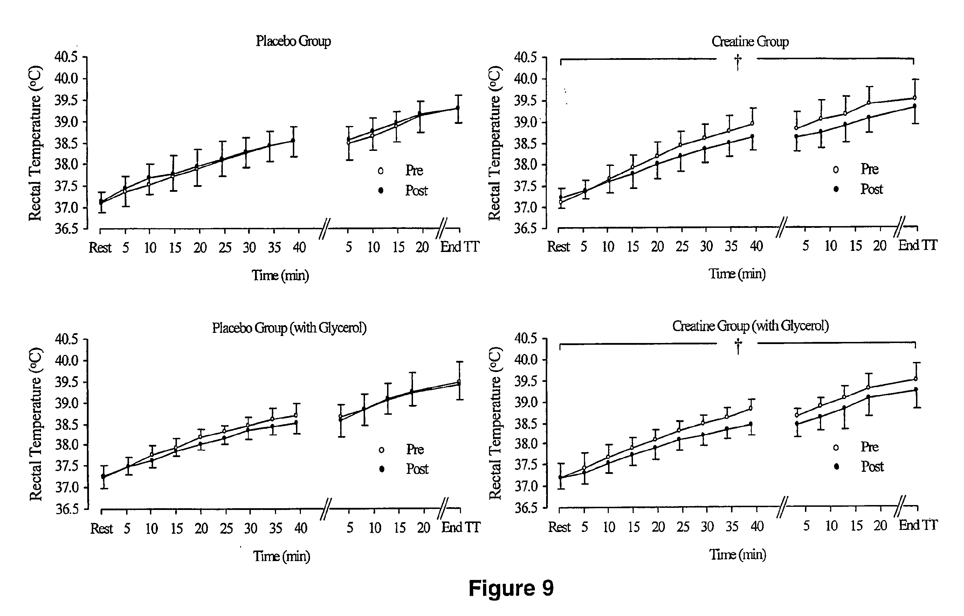

Core *Temperature.* Throughout the exercise period, T_{c} increased significantly during all trials (Figure 9). A simple main effects analysis revealed that T_{c} during exercise was not significantly different in the P1 group during either the P1 supplementation week or the Gly supplementation week (Figure 9). In the Cr group, T_{c} was significantly lower in both P1 (P=0.042) and Gly (P=0.021) supplementation weeks compared to pre-supplementation (Figure 9).

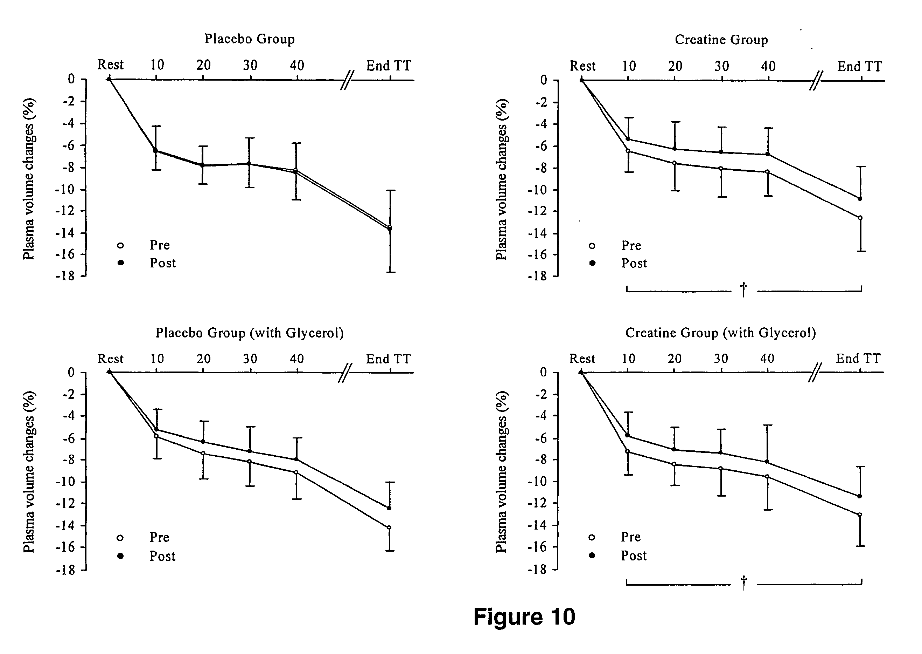

*Plasma volume (PV) changes.* Throughout the exercise period, PV decreased significantly during all trials. A simple main effects analysis and revealed that in the P1 group, ΔPV during exercise was not significantly different in the P1 group during either the P1 supplementation week or the Gly supplementation week (Figure 10). In the Cr group, there was a lower ΔPV post-supplementation compared to pre-supplementation in both P1 (P=0.029) and Gly (P=0.016) supplementation weeks (Figure 10).

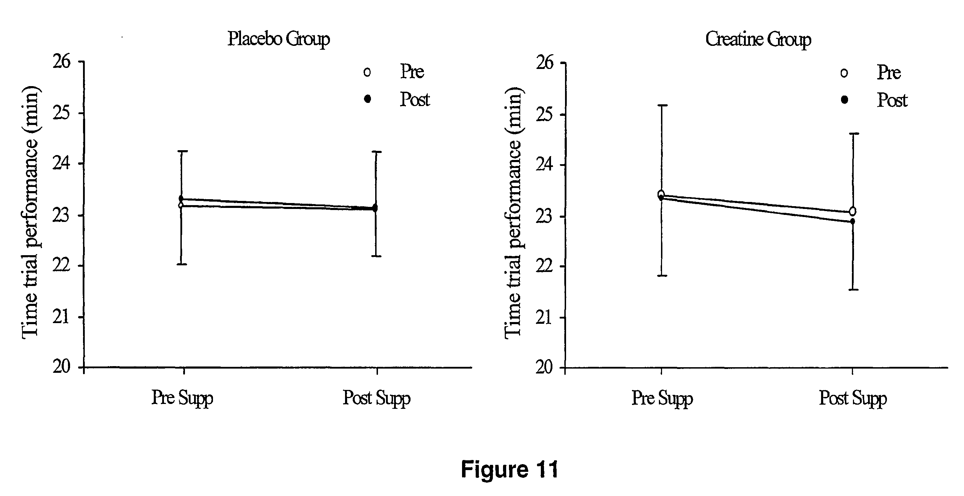

*Time Trial Performance.* Performance during the time trial was not significantly different between the groups prior to supplementation (P=0.41). Similarly, time trial performance was not influenced by supplementation in either the P1 group or Cr group (Figure 11).

*Side effects.* In general, subjects tolerated the supplementation protocol well, with only one report of gastrointestinal distress after Gly supplementation. 3 subjects from each group correctly identified the subject group and 2 subjects correctly identified the Gly supplementation week, while, all other subjects were unsure of the treatment they received.

*Other results.* The following data have also been collected/derived but not yet fully analysed: Estimated Cr uptake, weighted mean skin temperature, mean body temperature, metabolic rate, sweat rate, sweat loss, serum osmolality, blood glucose, blood lactate, and corrected ΔPV (i.e. corrected for changes in basal hydration levels).

### Discussion

This study has demonstrated that Gly, Cr and a combination of Cr and Gly increased BM, TBW and reduced cardiovascular and thermoregulatory responses during exercise in the heat. Furthermore, addition of Gly to a standard Cr supplementation regimen (Harris *et al.* 1992) further increased BM, TBW and reduced the cardiovascular response to exercise in the heat.

Gly supplementation in the current study attenuated the rise in HR during exercise in the heat (Figure 6), which is in agreement with previous findings by Anderson *et al.* (2001) and Montner *et al*. (1996). However, Gly supplementation had no effect on slowing the rise in T_{c} during the exercise period (Figure 9). Cr supplementation in the present study was successful in attenuating cardiovascular and thermoregulatory responses (i.e. decreased HR and T_{c} during exercise, Figure 6 and 9). This is consistent with previous studies examining the effects of Cr supplementation on physiological response to exercise in the heat (Kern *et al.* 2001, Kilduff *et al.* 2004). Addition of Gly to the Cr supplement significantly enhanced the attenuation in HR (Figure 6) but had no effect on T_{c} compared to Cr supplementation alone (Figure 9). Subjects who supplemented with Cr or Cr/Gly reported, on average, significantly lower ratings of perceived leg fatigue (Figure 7) and breathlessness (Figure 8), suggesting they were able to discern the benefit of these putative hyperhydration strategies.

Many hyperhydration methods have been tested during conditions of heat stress in an attempt to enhance thermoregulatory and cardiovascular responses, and subsequently, improve exercise performance. Despite some positive results (Anderson *et al*. 2001, Kilduff *et al*. 2004, Motner *et al*. 1996), these strategies have frequently failed to improve exercise performance (e.g. Latzka *et al*. 1997, Watt *et al.* 2000). For example, Watt *et al*. (2000) demonstrated that a 13% acute PV expansion, a level similar to that observed after heat acclimatisation, had no effect on T_{c}, skin blood flow, HR or exercise performance. These authors concluded that PV expansion might not be critical for changes in thermoregulation and exercise performance in the heat. It is somewhat surprising that despite the positive effects of Cr and the combined Cr/Gly supplementation on cardiovascular and thermoregulatory responses in the present study, there was no effect on 16.1 km time trial performance. This may be due to the fact that the time trial duration was too short for any benefits in thermoregulation to be discerned.

We have demonstrated that Gly, Cr and combined Cr/Gly supplementation can induce modest hyperhydration (Figure 4). Gly when consumed in combination with a substantial fluid intake, enhances the retention of this fluid and expands various body fluid spaces. Typically, this allows a fluid expansion or retention of ~600 ml above a fluid bolus alone, by reducing urinary volume. A review of glycerol as a hyperhydrating agent is provided by Robergs & Griffin (1998). In the present study, supplementation with Gly was successful in increasing TBW by an estimated 600 ml on average, of which 400 ml could be accounted for by the increase in ECW. On the other hand, Cr supplementation has previously been shown to increase TBW, and more specifically ICW. Despite this, the exact mechanism behind this increase remains unknown. Cr supplementation in the present study resulted in an increase in TBW by an estimated 680 ml on average, of which 500 ml could be accounted for by the increase in ICW. The increase in PV was approximately 51 ml assuming 7.5 % of the increase in TBW is plasma. Addition of Gly to the Cr supplementation had a tendency to further enhance the increase in TBW (and significantly enhance further the increase in BM). Combined Cr/Gly supplementation resulted in an increase in TBW by an estimated 900 ml, split evenly between intra- and extracellular water compartments (Table 4).

To date, the precise mechanism(s) that could attribute a more efficient thermoregulatory response during exercise in the heat, to an increase in TBW, remains largely unexplained. It is possible, however, that the various hyperhydration regimens in the present study may have resulted in an increase in the specific heat capacity of the body, resulting in greater capacity to store heat. For example, 0.83 kcal of heat production per kg of BM is required to increase T_{c} by 1° C; therefore an expansion of either ICW or ECW (and, hence, increase BM), could lead to a more efficient distribution of heat within the body. There are a number of published reports which lend support to this contention. For example, Kay & Marino (2000) proposed that fluid ingestion enhances exercise performance in the heat by increasing heat storage capacity of the body. Furthermore, in a review by Sawka (1992), it was suggested that a reduced heat storage capacity and all the consequent detrimental effects on exercise performance, may be incurred both as a direct result of deficits in intracellular and extracellular water compartments. The importance of maintaining intracellular water during exercise in the heat is highlighted in the work by Nose et al. (1988). These authors reported a strong association between loss of free water (sweat/urine) and the decrease in intracellular fluid following 90-100 min of exercise at 36° C, thus implicating a significant role for intracellular water in the sweating response, the latter of course a vital process for efficient thermoregulation in conditions of heat stress.

In the present study, combined Cr and Gly supplementation for 6 days was effective in increasing both ICW and ECW and reducing certain cardiovascular responses during prolonged exercise in the heat. The rise in heart rate, rectal temperature, and effort perception during exercise were significantly attenuated by Cr. Addition of Gly to Cr tended to increase TBW further than Cr alone (P=0.08) and significantly enhanced the attenuation in heart rate (P=0.02). These results show that a combination of Cr and Gly is a more effective hyperhydrating agent than either Cr or Gly alone.

While the invention has been described in conjunction with the exemplary embodiments described above, many equivalent modifications and variations will be apparent to those skilled in the art when given this disclosure.

### REFERENCES

1. American College of Sports Medicine (2000). Joint Position Statement: nutrition and athletic performance. American College of Sports Medicine, American Dietetic Association, and Dietitians of Canada. Med Sci Sports Exerc. 32, 2130-2145.
2. Anderson MJ, Cotter JD, Garnham AP, Casley DJ, Febbraio MA (2001). Effect of glycerol-induced hyperhydration on thermoregulation and metabolism during exercise in the heat. Int J Sport Nutr Exerc Metab 11, 315-333.
3. Armstrong LE, Hubbard RW, Jones BH, Daniels JJ (1986). Preparing Alberto Salazar for the heat of the 1984 Olympic marathon. Physician Sportsmed 14, 73-81.
4. Beaver WL, Wasserman K, Whipp BJ (1986) A new method for detecting anaerobic threshold by gas exchange. J Appl Physiol 60: 2020-2027.
5. Boobis LH, Maughan RJ (1983) A simple one-step enzymatic fluorimetric method for the determination of glycerol in 20 ml of plasma. Clin Chim Acta 132, 173-179
6. Borg GA (1982) Psychophysical bases of perceived exertion. Med Sci Sports Exerc. 14, 377-381.
7. Casey A, Constantin-Teodosiu D, Howell S, Hultman E, Greenhaff PL (1996). Creatine supplementation favourably affects performance and muscle metabolism during maximal intensity exercise in humans. Am J Physiol 271, E31-E37.
8. Dill DB, Costill DL (1974) Calculation of percentage changes in volumes of blood, plasma, and red cells in dehydration. J Appl Physiol 37, 247-248.
9. Forster HV, Dempsey JA, Thomson J, Vidruk R, DoPico G A (1972) Estimation of arterial PO2, PCO2, pH and lactate from arterialised venous blood. J Appl Physiol 32, 134-137.
10. Francaux M, Poortmans JR (1999). Effects of training and creatine supplement on muscle strength and body mass. Eur J Appl Physiol 80, 165-168.
11. Galloway SDR & Maughan RJ (1997). Effects of ambient temperature on the capacity to perform prolonged cycle exercise in man. Med Sci Sports Exerc 29, 1240-1249.
12. Gisolfi CV, Summers RW, Schedl HP, Bleiler TL, Oppliger RA (1990). Human intestinal water absorption: direct vs. indirect measurements. Am J Physiol. 258(2 Pt 1), G216-G222.
13. Gisolfi CV, Spranger KJ, Summers RW, Schedel HP, Bleiler TL (1991). Efects of cycle exercise on intestinal absorption in humans. J Appl Physiol 71, 2518-2527.
14. Gleeson M, Maughan RJ, Greenhaff PL (1986). Comparison of the effects of pre-exercise feeding of glucose, glycerol, and placebo on endurance and fuel homeostasis in man. Eur J Appl Physiol 55, 645-53.
15. Greenleaf JE, Castle BL (1971). Exercise temperature regulation in man during hypohydration and hyperhydration. J Appl Physiol 30, 847-853.
16. Harris RC, Sodehind K, Hultman E (1992) Elevation of Creatine in resting and exercised muscle of normal subjects by Cr supplementation. Clin Sci 83, 367-374.
17. Hitchins S, Martin DT, Dobson GP, Burke L, Yates K, Hahn A (1999). Glycerol hyperhydtration improves cycle time trial performance in hot humid conditions. Eur J Appl Physiol 80, 494-501.
18. Holland, B., Welch, A.A., Unwin, I.D., Buss, D.H., Paul, A.A. & Southgate, D.A.T. (1991). The composition of foods. 5th edn; Cambridge: Goodfellow Egan Phototypesetting Ltd.
19. Kern M, Podewils LJ, Vukovich M, Buono MJ (2001) Physiological response to exercise in the heat following Creatine supplementation. JEPonline 4, 18-27.
20. Kilduff LP, Georgiades E, James N, Minnion RH, Mitchell M, Kingsmore D, Hadjicharlambous M, Pitsiladis YP (2004). The effects of creatine supplementation on cardiovascular, metabolic, and thermoregulatory responses during exercise in the heat in endurance-trained humans. Int J Sport Nutr Exerc Metab 14, 443-60.
21. Kilduff LP, Vidakovic P, Cooney G, Twycross-Lewis R, Amuna P, Parker M, Paul L, Pitsiladis YP (2002). Effects of creatine on isometric bench-press performance in resistance-trained humans. Med Sci Sports Exerc 34, 1176-1183.
22. Kay D, Marino FE (2000). Fluid ingestion and exercise hyperthermia: implications for performance, thermoregulation, metabolism and the development of fatigue. J Sports Sci 18, 71-82.
23. Kreider R, Ferreira M, Wilson M, Grindstaff P, Plisk S, Reinardy J, Cantler E, Almanda A (1998). Effects of creatine supplementation on body composition, strength, and sprint performance. Med Sci Sports Exerc 1, 73-82.
24. Latzka WA, Sawka MN, Montain SJ, Skrinar GS, Fielding RA, Matott RP, Pandolf KB (1997) Hyperhydration: thermoregulatory effects during compensable exercise-heat stress. J. Appl. Physiol. 83(3), 860-866.
25. Latzka WA, Sawka MN, Montain SJ, Skrinar GS, Fielding RA, Matott RP, Pandolf KB (1998) Hyperhydration: tolerance and cardiovascular effects during uncompensable exercise-heat stress. J Appl Physiol. 84(6):1858-1864.
26. Lyons TP, Riedesel ML, Meuli LE, Chick TW (1990). Effects of glycerol induced hyperhydration prior to exercise in the heat on sweating and core temperature. Med Sci Sports Exerc 22, 77-83.
27. Marino FE, Kay D, Cannon J (2003) Glycerol hyperhydration fails to improve endurance performance and thermoregulation in humans in a warm humid environment. Pflugers Arch. 446(4):455-462.
28. McCance, RA, Widdowson ED (1991). The composition of foods. (Eds.) 5th revised and extended ed. Cambridge: Goodfellow Egan Phototypesetting Ltd.
29. Mitchell JW, Nadel ER, Stolwijk JAJ (1972) Respiratory weight losses during exercise. J Appl Physiol 32, 474-476.
30. Montner P, Stark DM, Riedesel MC, Murata G, Robergs R, Timms M, Chick TW (1996). Pre-exercise glycerol hydration improves cycling endurance time. Int J Sports Med 17, 27-33.
31. Moroff SV, Bass DE (1965). Effect of overhydration on man's physiological responses to work in the heat. J Appl Physiol 20, 267-270.
32. Nadel ER, Fortney SM, Wenger CB (1980). Effect of hydration state on circulatory and thermal regulations. J Appl Physiol 49, 715-721.
33. Noakes, TD (2003). `Position statement. Fluid replacement during marathon running', Clin J Sports Med. 13, 309-18.
34. Nose H, Mack GW, Shi XR, Nadel ER (1988). Shift in body fluid compartments after dehydration in humans. J Appl Physiol 65, 318-324.
35. Olschewski H, Bruck K (1988) Thermoregulatory, cardiovascular, and muscular factors related to exercise after precooling. J Appl Physiol 64, 803-811.
36. Ramanthan NL (1964) A new weighting system for mean surface temperature of the human body. J Appl Physiol 19, 531-533.
37. Ravussin E, Schutz Y, Acheson KJ, Dusmet M, Bourquin L, Jequier E (1985) Short-term, mixed-diet overfeeding in man: no evidence of "luxuskonsumption". Am J Physiol 249, E470-E477.
38. Robergs RA, Griffin SE (1998). Glycerol: biochemistry, phar-macokinetics and clinical and practical applications. Sports Med 26, 145 -167.
39. Saltin B (1964). Circulatory response to submaximal and maximal exercise after thermal dehydration. J Appl Physiol 19, 1125-1132.
40. Sawka MN (1992). Physiological consequences of hypohydration: exercise performance and thermoregulation. Med Sci Sports Ex 24, 67-670.
41. Vandenberghe K, Gillis N, Van Leemputte M, Van Hecke P, Vanstapel F, Hespel P (1996). Caffeine counteracts the ergogenic action of muscle creatine loading. J Appl Physiol. 80(2), 452-457.
42. Van Loan MD (1990) Bioelectrical impedance analysis to determine fat-free mass, total body water and body fat. Sports Med. 10, 205-217.
43. Watt MJ, Garnham AP, Febbraio MA, Hargreaves M (2000). Effect of acute plasma volume expansion on thermoregulation and exercise performance in the heat, Med. Sci. Sports Exerc. 32, 958-962.

## Claims

1. A non-therapeutic method of increasing the capacity of a subject to absorb ingested water and/or to retain body water, comprising administering creatine and glycerol to the subject.

2. A method according to claim 1 comprising the step of administering glycerol to the subject at least once per day for at least two days, at least five days, or at least 10 days.

3. A method according to claim 2 wherein each administration provides a dose of between about 0.1 g and about 2 g glycerol per kg body weight.

4. A method according to claim 3 wherein each administration provides a dose of between about 0.3 g and about 1.0 g glycerol per kg body weight.

5. A method according to claim 4 wherein each administration provides a dose of about 0.5 g or about 1 g glycerol per kg body weight.

6. A method according to claim 2 wherein each administration provides a dose of between about 7 g and about 140 g of glycerol.

7. A method according to claim 6 wherein each administration provides a dose of between about 21 g and about 70 g glycerol.

8. A method according to claim 7 wherein each administration provides a dose of about 37.5 g or about 70 g glycerol.

9. A method according to claim 2 wherein up to about 4 g of glycerol is administered per kg body weight per day.

10. A method according to claim 9 wherein between about 0.5 g and about 2 g of glycerol is administered per kg per day.

11. A method according to claim 2 wherein between about 7 g and about 280 g glycerol is administered per day.

12. A method according to claim 11 wherein between about 21 g and about 210 g glycerol is administered per day.

13. A method according to claim 12 wherein between about 37.5 g and about 140 g glycerol is administered per day.

14. A method according to claim 2 wherein a first quantity of glycerol is administered daily for a conditioning period, and a second quantity of glycerol is administered daily for a subsequent maintenance period, wherein the first quantity is larger than the second quantity.

15. A method according to claim 14 wherein the conditioning period is from one to fourteen days.

16. A method according to claim 15 wherein the conditioning period is from three to ten days.

17. A method according to any one of claims 14 to 16 wherein glycerol is administered two, three or four times per day during the conditioning period and once per day during the maintenance period.

18. A method according to claim 17 wherein the quantity of glycerol administered daily during the conditioning period is between about two and about five times greater than the quantity administered daily during the maintenance period.

19. A method according to claim 18 therein about 2 g glycerol per kg body weight is administered per day for the conditioning period, followed by about 0.5 g per kg body weight per day during the maintenance period.

20. A method according to claim 18 wherein about 140 g glycerol is administered per day for the conditioning period, and about 37.5 g glycerol per day during the maintenance period.

21. A method according to any one of the preceding claims comprising the step of administering creatine to the subject at least once per day for at least two days.

22. A method according to claim 21 wherein each individual administration comprises a dose of at least 0.5 g, at least 3 g, at least 5 g, or at least 10 g of creatine.

23. A method according to claim 22 wherein between about 3 g and about 30 g of creatine is administered per day.

24. A method according to claim 23 wherein between about 5 g and about 20 g of creatine is administered per day.

25. A method according to claim 21 wherein a first quantity of creatine is administered daily for a conditioning period, and a second quantity of creatine is administered daily for a subsequent maintenance period, wherein the first quantity is larger than the second quantity.

26. A method according to claim 25 wherein the conditioning period is from one to fourteen days.

27. A method according to claim 26 wherein the conditioning period is from three to ten days.

28. A method according to any one of claims 25 to 27 wherein creatine is administered two, three or four times per day during the conditioning period and once per day during the maintenance period.

29. A method according to claim 28 wherein the quantity of creatine administered daily during the conditioning period is between about two and about five times greater than the quantity administered daily during the maintenance period.

30. A method according to claim 29 wherein about 20 g of creatine is administered per day during the conditioning period and about 5 g of creatine during the maintenance period.

31. A method according to anyone of the preceding claims further comprising the step of administering carbohydrate to the subject.

32. A method according to claim 31 wherein the carbohydrate is administered in the same composition as the glycerol and the creatine,

33. A method according to claim 31 or claim 32 wherein the carbohydrate is glucose polymer.

34. A method according to any one of claims 31 to 33 wherein each individual administration provides at least about 30 g of glucose.

35. A composition comprising creatine and glycerol for use in a method of treating dehydration, heat stroke, heat cramps or heat exhaustion, optionally in conjunction with carbohydrate.

36. A composition according to claim 35 wherein the medicament or composition further comprises carbohydrate.

37. A composition according to claim 36 wherein the carbohydrate is glucose polymer.

38. A composition according to any one of claims 35 to 37, wherein about 20 parts by weight of glycerol are present per part by weight of creatine.

39. A composition according to claim 38 wherein about 5 and about 10 parts glycerol are present per part creatine.

40. A composition according to any one of claims 35 to 39, wherein the composition or medicament is suitable for oral or intravenous administration.

41. Use of creatine in the manufacture of a medicament or composition for the prevention or treatment of dehydration, heat stroke, heat cramps or heat exhaustion , wherein the medicament or composition is for administration in conjunction with glycerol.

42. Use of glycerol in the manufacture of a medicament or composition for the prevention or treatment of dehydration, heat stroke, heat cramps or heat exhaustion, wherein the medicament or composition is for administration in conjunction with creatine,

43. Use of creatine and glycerol in the manufacture of a medicament or composition for the prevention or treatment of dehydration, heat stroke, heat cramps or heat exhaustion.

44. Use according to any one of claims 41 to 43, wherein the medicament or composition further comprises carbohydrate.

45. Use according to claim 44 wherein the carbohydrate is glucose polymer.

46. Use according to any one of claims 41 to 45, wherein about 20 parts by weight of glycerol are present per part by weight of creatine.

47. Use according to claim 46 wherein about 5 and about 10 parts glycerol are present per part creatine.

48. Use according to any one of claims 41 to 47, wherein the composition or medicament is suitable for oral or intravenous administration.

## Patentansprüche

1. Nichttherapeutisches Verfahren zur Steigerung der Fähigkeit eines Individuums, aufgenommenes Wasser zu absorbieren und/oder Körperwasser zu halten, umfassend die Verabreichung von Creatin und Glycerin an das Individuum.

2. Verfahren nach Anspruch 1, umfassend den Schritt der Verabreichung von Glycerin an das Individuum zumindest 1-mal pro Tag über zumindest 2 Tage, zumindest 5 Tage oder zumindest 10 Tage hinweg.

3. Verfahren nach Anspruch 2, worin jede Verabreichung eine Dosis zwischen etwa 0,1 g und etwa 2 g Glycerin pro kg Körpergewicht bereitstellt.

4. Verfahren nach Anspruch 3, worin jede Verabreichung eine Dosis zwischen etwa 0,3 g und etwa 1,0 g Glycerin pro kg Körpergewicht bereitstellt.

5. Verfahren nach Anspruch 4, worin jede Verabreichung eine Dosis von etwa 0,5 g oder etwa 1 g Glycerin pro kg Körpergewicht bereitstellt.

6. Verfahren nach Anspruch 2, worin jede Verabreichung eine Dosis zwischen etwa 7 g und etwa 140 g Glycerin bereitstellt.

7. Verfahren nach Anspruch 6, worin jede Verabreichung eine Dosis zwischen etwa 21 g und etwa 70 g Glycerin bereitstellt.

8. Verfahren nach Anspruch 7, worin jede Verabreichung eine Dosis von etwa 37,5 g oder etwa 70 g Glycerin bereitstellt.

9. Verfahren nach Anspruch 2, worin bis zu etwa 4 g Glycerin pro kg Körpergewicht pro Tag verabreicht werden.

10. Verfahren nach Anspruch 9, worin zwischen etwa 0,5 g und etwa 2 g Glycerin pro kg pro Tag verabreicht werden.

11. Verfahren nach Anspruch 2, worin zwischen etwa 7 g und etwa 280 g Glycerin pro Tag verabreicht werden.

12. Verfahren nach Anspruch 11, worin zwischen etwa 21 g und etwa 210 g Glycerin pro Tag verabreicht werden.

13. Verfahren nach Anspruch 12, worin zwischen etwa 37,5 g und etwa 140 g Glycerin pro Tag verabreicht werden.

14. Verfahren nach Anspruch 2, worin in einer Konditionierungsphase täglich eine erste Menge Glycerin verabreicht wird und in einer darauf folgenden Erhaltungsphase täglich eine zweite Menge Glycerin verabreicht wird, wobei die erste Menge größer ist als die zweite Menge.

15. Verfahren nach Anspruch 14, worin die Konditionierungsphase 1 bis 14 Tage umfasst.

16. Verfahren nach Anspruch 15, worin die Konditionierungsphase 3 bis 10 Tage umfasst.

17. Verfahren nach einem der Ansprüche 14 bis 16, worin das Glycerin während der Konditionierungsphase 2-, 3- oder 4-mal pro Tag und während der Erhaltungsphase 1-mal pro Tag verabreicht wird.

18. Verfahren nach Anspruch 17, worin die während der Konditionierungsphase täglich verabreichte Glycerinmenge zwischen etwa 2- und etwa 5-mal höher ist als die während der Erhaltungsphase täglich verabreichte Menge.

19. Verfahren nach Anspruch 18, worin während der Konditionierungsphase etwa 2 g Glycerin pro kg Körpergewicht pro Tag verabreicht werden, worauf während der Erhaltungsphase etwa 0,5 g pro kg Körpergewicht pro Tag folgen.

20. Verfahren nach Anspruch 18, worin während der Konditionierungsphase etwa 140 g Glycerin pro Tag und während der Erhaltungsphase etwa 37,5 g Glycerin pro Tag verabreicht werden.

21. Verfahren nach einem der vorangegangenen Ansprüche, umfassend den Schritt des Verabreichens von Creatin an das Individuum zumindest 1-mal pro Tag über zumindest 2 Tage hinweg.

22. Verfahren nach Anspruch 21, worin jede Verabreichung eine Dosis von zumindest 0,5 g, zumindest 3 g, zumindest 5 g oder zumindest 10 g Creatin umfasst.

23. Verfahren nach Anspruch 22, worin zwischen etwa 3 g und etwa 30 g Creatin pro Tag verabreicht werden.

24. Verfahren nach Anspruch 23, worin zwischen etwa 5 g und etwa 20 g Creatin pro Tag verabreicht werden.

25. Verfahren nach Anspruch 21, worin während einer Konditionierungsphase eine erste Menge Creatin täglich verabreicht wird und während einer darauf folgenden Erhaltungsphase eine zweite Menge Creatin täglich verabreicht wird, wobei die erste Menge größer ist als die zweite Menge.

26. Verfahren nach Anspruch 25, worin die Konditionierungsphase 1 bis 14 Tage umfasst.

27. Verfahren nach Anspruch 26, worin die Konditionierungsphase 3 bis 10 Tage umfasst.

28. Verfahren nach einem der Ansprüche 25 bis 27, worin Creatin während der Konditionierungsphase 2-, 3- oder 4-mal pro Tag und während der Erhaltungsphase 1-mal pro Tag verabreicht wird.

29. Verfahren nach Anspruch 28, worin die während der Konditionierungsphase täglich verabreichte Creatinmenge zwischen etwa 2- und etwa 5-mal höher ist als die während der Erhaltungsphase täglich verabreichte Menge.

30. Verfahren nach Anspruch 29, worin während der Konditionierungsphase etwa 20 g pro Tag Creatin und während der Erhaltungsphase etwa 5 g Creatin pro Tag verabreicht werden.

31. Verfahren nach einem der vorangegangenen Ansprüche, weiters umfassend den Schritt der Verabreichung von Kohlenhydraten an das Individuum.

32. Verfahren nach Anspruch 31, worin die Kohlenhydrate in derselben Zusammensetzung wie das Glycerin und das Creatin verabreicht werden.

33. Verfahren nach Anspruch 31 oder 32, worin die Kohlenhydrate ein Glucosepolymere sind.

34. Verfahren nach einem der Ansprüche 31 bis 33, worin jede einzelne Verabreichung zumindest etwa 30 g Glucose bereitstellt.

35. Zusammensetzung, die Creatin und Glycerin umfasst, zur Verwendung in einem Verfahren zur Behandlung von Dehydrierung, Hitzeschlag, Hitzekrämpfen oder Hitzeerschöpfung, gegebenenfalls in Verbindung mit Kohlenhydraten.

36. Zusammensetzung nach Anspruch 35, worin das Medikament oder die Zusammensetzung weiters Kohlenhydrate umfasst.

37. Zusammensetzung nach Anspruch 36, worin die Kohlenhydrate Glucosepolymere sind.

38. Zusammensetzung nach einem der Ansprüche 35 bis 37, worin etwa 20 Gewichtsteile Glycerin pro Gewichtsteil Creatin vorhanden sind.

39. Zusammensetzung nach Anspruch 38, worin zwischen etwa 5 und etwa 10 Gewichtsteile Glycerin pro Gewichtsteil Creatin vorhanden sind.

40. Zusammensetzung nach einem der Ansprüche 35 bis 39, worin die Zusammensetzung oder das Medikament zur oralen oder intravenösen Verabreichung geeignet ist.

41. Verwendung von Creatin zur Herstellung eines Medikaments oder einer Zusammensetzung zur Prävention oder Behandlung von Dehydrierung, Hitzeschlag, Hitzekrämpfen oder Hitzeerschöpfung, wobei das Medikament oder die Zusammensetzung zur Verabreichung in Verbindung mit Glycerin gedacht ist.

42. Verwendung von Glycerin zur Herstellung eines Medikaments oder einer Zusammensetzung zur Prävention oder Behandlung von Dehydrierung, Hitzeschlag, Hitzekrämpfen oder Hitzeerschöpfung, wobei das Medikament oder die Zusammensetzung zur Verabreichung in Verbindung mit Creatin gedacht ist.

43. Verwendung von Creatin und Glycerin zur Herstellung eines Medikaments oder einer Zusammensetzung zur Prävention oder Behandlung von Dehydrierung, Hitzeschlag, Hitzekrämpfen oder Hitzeerschöpfung.

44. Verwendung nach einem der Ansprüche 41 bis 43, wobei das Medikament oder die Zusammensetzung weiters Kohlenhydrate umfasst.

45. Verwendung nach Anspruch 44, wobei die Kohlenhydrate Glucosepolymere sind.

46. Verwendung nach einem der Ansprüche 41 bis 45, wobei etwa 20 Gewichtsteile Glycerin pro Gewichtsteil Creatin vorhanden sind.

47. Verwendung nach Anspruch 46, wobei zwischen etwa 5 und etwa 10 Gewichtsteile Glycerin pro Gewichtsteil Creatin vorhanden sind.

48. Verwendung nach einem der Ansprüche 41 bis 47, wobei die Zusammensetzung oder das Medikament zur oralen oder intravenösen Verabreichung geeignet ist.

## Revendications

1. Procédé non-thérapeutique pour augmenter la capacité d'un sujet à absorber de l'eau ingérée et/ou à retenir l'eau dans le corps, comprenant l'administration de créatine et de glycérol au sujet.

2. Procédé selon la revendication 1, comprenant l'étape consistant à administrer du glycérol au sujet au moins une fois par jour pendant au moins deux jours, au moins cinq jours, ou au moins 10 jours.

3. Procédé selon la revendication 2, où chaque administration prévoit une dose entre environ 0,1 g et environ 2 g de glycérol par kg de poids corporel.

4. Procédé selon la revendication 3, où chaque administration prévoit une dose entre environ 0,3 g et environ 1,0 g de glycérol par kg de poids corporel.

5. Procédé selon la revendication 4, où chaque administration prévoit une dose d'environ 0,5 g ou d'environ 1 g de glycérol par kg de poids corporel.

6. Procédé selon la revendication 2, où chaque administration prévoit une dose entre environ 7g et environ 140g de glycérol.

7. Procédé selon la revendication 6, où chaque administration prévoit une dose entre environ 21 g et environ 70 g de glycérol.

8. Procédé selon la revendication 7, où chaque administration prévoit une dose d'environ 37,5 g ou environ 70 g de glycérol.

9. Procédé selon la revendication 2, où jusqu'à environ 4 g de glycérol sont administrés par kg de poids corporel par jour.

10. Procédé selon la revendication 9, où entre environ 0,5 et environ 2 g de glycérol sont administrés par kg par jour.

11. Procédé selon la revendication 2, où entre environ 7 g et environ 280 g de glycérol sont administrés par jour.

12. Procédé selon la revendication 11, où entre environ 21 g et environ 210 g de glycérol sont administrés par jour.

13. Procédé selon la revendication 12, où entre environ 37,5 g et environ 140 g de glycérol sont administrés par jour.

14. Procédé selon la revendication 2, où une première quantité de glycérol est administrée quotidiennement durant une période de conditionnement, et une deuxième quantité de glycérol est administrée quotidiennement durant une période de maintient suivante, où la première quantité est plus grande que la deuxième quantité.

15. Procédé selon la revendication 14, où la période de conditionnement est d'un à quatorze jours.

16. Procédé selon la revendication 15, où la période de conditionnement est de trois à dix jours.

17. Procédé selon l'une quelconque des revendications 14 à 16, où le glycérol est administré deux, trois ou quatre fois par jour durant la période de conditionnement et une fois par jour durant la période de maintien.

18. Procédé selon la revendication 17, où la quantité de glycérol administrée quotidiennement durant la période de conditionnement est entre environ deux et environ cinq fois plus grande que la quantité administrée quotidiennement durant la période de maintien.

19. Procédé selon la revendication 18, où environ 2 g de glycérol par kg de poids corporel est administré par jour durant la période de conditionnement, suivi d'environ 0,5 g par kg de poids corporel par jour durant la période de maintien.

20. Procédé selon la revendication 18, où environ 140 g de glycérol sont administrés par jour durant la période de conditionnement, et environ 37,5 g de glycérol par jour durant la période de maintien.

21. Procédé selon l'une quelconque des revendications précédentes, comprenant l'étape consistant à administrer de la créatine au sujet au moins une fois par jour pendant au moins deux jours.

22. Procédé selon la revendication 21, où chaque administration individuelle comprend une dose d'au moins 0,5 g, d'au moins 3 g, d'au moins 5 g, ou d'au moins 10 g de créatine.

23. Procédé selon la revendication 22, où entre environ 3 g et environ 30 g de créatine sont administrés par jour.

24. Procédé selon la revendication 23, où entre environ 5 g et environ 20 g de créatine sont administrés par jour.

25. Procédé selon la revendication 21, où une première quantité de créatine est administrée quotidiennement durant une période de conditionnement, et une deuxième quantité de créatine est administrée quotidiennement durant une période de maintien suivante, où la première quantité est plus grande que la deuxième quantité.

26. Procédé selon la revendication 25, où la période de conditionnement est d'un à quatorze jours.

27. Procédé selon la revendication 26, où la période de conditionnement est de trois à dix jours.

28. Procédé selon l'une quelconque des revendications 25 à 27, où la créatine est administrée deux, trois ou quatre fois par jour durant la période de conditionnement et une fois par jour durant la période de maintien.

29. Procédé selon la revendication 28, où la quantité de créatine administrée quotidiennement durant la période de conditionnement est entre environ deux et environ cinq fois plus grande que la quantité administrée quotidiennement durant la période de maintien.

30. Procédé selon la revendication 29, où environ 20 g de créatine est administré par jour durant la période de conditionnement et environ 5 g de créatine durant la période de maintien.

31. Procédé selon l'une quelconque des revendications précédentes, comprenant en outre l'étape consistant à administrer de l'hydrate de carbone au sujet.

32. Procédé selon la revendication 31, où l'hydrate de carbone est administré dans la même composition que le glycérol et la créatine.

33. Procédé selon la revendication 31 ou la revendication 32, où l'hydrate de carbone est un polymère de glucose.

34. Procédé selon l'une quelconque des revendications 31 à 33, où chaque administration individuelle fournit au moins environ 30 g de glucose.

35. Composition comprenant de la créatine et du glycérol pour utilisation dans un procédé pour traiter la déshydratation, un coup de chaleur, des crampes de chaleur ou l'épuisement dû à la chaleur, optionnellement conjointement avec un hydrate de carbone.

36. Composition selon la revendication 35, où le médicament ou la composition comprend en outre un hydrate de carbone.

37. Composition selon la revendication 36, où l'hydrate de carbone est un polymère de glucose.

38. Composition selon l'une quelconque des revendications 35 à 37, où environ 20 parts en poids de glycérol sont présents par parties en poids de créatine.

39. Composition selon la revendication 38, où environ 5 et environ 10 parties de glycérol sont présentes par parties de créatine.

40. Composition selon l'une quelconque des revendications 35 à 39, où la composition ou médicament convient pour l'administration orale ou intraveineuse.

41. Utilisation de la créatine dans la fabrication d'un médicament ou d'une composition pour empêcher ou traiter la déshydratation, un coup de chaleur, des crampes de chaleur, ou l'épuisement dû à la chaleur, où le médicament ou la composition est pour l'administration conjointement avec du glycérol.

42. Utilisation de glycérol dans la fabrication d'un médicament ou d'une composition pour empêcher ou traiter la déshydratation, un coup de chaleur, des crampes de chaleur ou un épuisement dû à la chaleur, où le médicament ou la composition est pour l'administration conjointement avec la créatine.

43. Utilisation de créatine et de glycérol dans la fabrication d'un médicament ou d'une composition pour empêcher ou traiter la déshydratation, un coup de chaleur, des crampes de chaleur ou un épuisement dû à la chaleur.

44. Utilisation selon l'une quelconque des revendications 41 à 43, où le médicament ou la composition comprend en outre un hydrate de carbone.

45. Utilisation selon la revendication 44, où l'hydrate de carbone est du polymère de glucose.

46. Utilisation selon l'une quelconque des revendications 41 à 45, où environ 20 parties en poids de glycérol sont présentes par parties en poids de créatine.

47. Utilisation selon la revendication 46, où environ 5 à environ 10 parties de glycérol sont présentes par parties de créatine.

48. Utilisation selon l'une quelconque des revendications 41 à 47, où la composition ou le médicament convient pour l'administration orale ou intraveineuse.
